# EUROPEAN PATENT SPECIFICATION

(11) **EP 3 368 028 B1**
(45) Date of publication and mention of the grant of the patent: **11.12.2024**
(21) Application number: 16794819.9
(22) Date of filing: 26.10.2016
(51) Int. Cl.: A61K 31/19, A61P 35/00, A61P 1/00, A61K 31/198, A61K 9/48, A61K 49/00, A61K 9/20, A61K 9/107, A61K 9/08, A61P 1/04, A61P 1/18, A61P 9/10, A61P 11/00, A61P 19/02, A61K 9/10, A61K 47/59, A61P 25/00

(54) **DENDERIMER COMPOSITIONS AND USE IN TREATMENT OF NECROTIZING ENTEROCOLITIS AND OTHER GASTROINTESTINAL DISORDERS**
DENDRIMERZUSAMMENSETZUNGEN UND VERWENDUNG ZUR BEHANDLUNG VON NEKROTISIERENDER ENTEROKOLITIS UND ANDEREN GASTROINTESTINALEN ERKRANKUNGEN
COMPOSITIONS ET UTILISATION DE DENDRIMÈRES DANS LE TRAITEMENT DE L'ENTÉROCOLITE NÉCROSANTE ET AUTRES TROUBLES GASTRO-INTESTINAUX

(30) Priority: 29.10.2015 US 201562248063 P
(43) Date of publication of application: 05.09.2018
(73) Proprietor: The Johns Hopkins University, Baltimore, MD 21218 (US)
(72) Inventor: HACKAM, David, Baltimore, MD 21208 (US); KANNAN, Sujatha, Highland, MD 20777 (US); RANGARAMANUJAM, Kannan, Highland, MD 20777 (US); NINO, Diego, F., Baltimore, MD 21208 (US); ZHANG, Fan, Baltimore, MD 21218 (US)
(74) Representative: Potter Clarkson
(86) International application number: PCT/US2016/058763
(87) International publication number: WO 2017/074993

(56) References cited:
- WO-A1-2015/038493
- US-A1- 2003 180 250
- TEO IAN ET AL: "Preventing acute gut wall damage in infectious diarrhoeas with glycosylated dendrimers.", EMBO MOLECULAR MEDICINE, vol. 4, no. 9, September 2012 (2012-09-01), pages 866 - 881, XP002765410, ISSN: 1757-4684
- ISLAM DILARA ET AL: "Controlling the cytokine storm in severe bacterial diarrhoea with an oral Toll-like receptor 4 antagonist.", IMMUNOLOGY, vol. 147, no. 2, February 2016 (2016-02-01), pages 178 - 189, XP002765411, ISSN: 1365-2567
- S. KANNAN ET AL: "Dendrimer-Based Postnatal Therapy for Neuroinflammation and Cerebral Palsy in a Rabbit Model", SCIENCE TRANSLATIONAL MEDICINE, vol. 4, no. 130, 18 April 2012 (2012-04-18), pages 1 - 13, XP055330569, ISSN: 1946-6234, DOI: 10.1126/scitranslmed.3003162
- NAVATH RAGHAVENDRA S ET AL: "Dendrimer-drug conjugates for tailored intracellular drug release based on glutathione levels.", BIOCONJUGATE CHEMISTRY, vol. 19, no. 12, December 2008 (2008-12-01), pages 2446 - 2455, XP002765412, ISSN: 1520-4812
- MISHRA MANOJ K ET AL: "Dendrimer brain uptake and targeted therapy for brain injury in a large animal model of hypothermic circulatory arrest.", ACS NANO, vol. 8, no. 3, 25 March 2014 (2014-03-25), pages 2134 - 2147, XP002765413, ISSN: 1936-086X
- YIYUN C ET AL: "Polyamidoamine dendrimers used as solubility enhancers of ketoprofen", EUROPEAN JOURNAL OF MEDICINAL CHEMISTRY, vol. 40, no. 12, 1 December 2005 (2005-12-01), EDITIONS SCIENTIFIQUE ELSEVIER, PARIS, FR, pages 1390 - 1393, XP027857630, ISSN: 0223-5234, [retrieved on 20051201]
- S. SADEKAR ET AL: "Transepithelial transport and toxicity of PAMAM dendrimers: Implications for oral drug delivery", ADVANCED DRUG DELIVERY REVIEWS, vol. 64, no. 6, 1 May 2012 (2012-05-01), pages 571 - 588, XP055054178, ISSN: 0169-409X, DOI: 10.1016/j.addr.2011.09.010
- NEAL MATTHEW D ET AL: "Discovery and validation of a new class of small molecule Toll-like receptor 4 (TLR4) inhibitors.", PLOS ONE, vol. 8, no. 6, E65779, June 2013 (2013-06-01), pages 1 - 10, XP002765414, ISSN: 1932-6203
- FANG MIN ET AL: "Host-guest chemistry of dendrimer-drug complexes: 7. Formation of stable inclusions between acetylated dendrimers and drugs bearing multiple charges.", THE JOURNAL OF PHYSICAL CHEMISTRY. B, vol. 116, no. 10, 15 March 2012 (2012-03-15), pages 3075 - 3082, XP002765415, ISSN: 1520-5207

## Description

### CROSS-REFERENCE TO RELATED APPLICATIONS

This application claims priority to U.S.S.N. 62/248,063, filed on October 29, 2015.

### FIELD OF THE INVENTION

This invention relates to oral formulations of poly(amidoamine) dendrimers for the treatment and/or diagnosis of inflammatory disorders such as necrotizing enterocolitis.

### BACKGROUND OF THE INVENTION

Necrotizing enterocolitis (NEC) is a severe inflammatory condition that affects the gastrointestinal tract of premature infants, and is characterized by the sudden development of intestinal necrosis followed by systemic sepsis and death in over 40% of cases. In those children who survive the onset of NEC, approximately 15% develop severe neurological injury, which is characterized by severely impaired cognition. Systemic inflammation, and neuroinflammation are the key known consequences associated with NEC.

Necrotizing enterocolitis is an acquired disease, primarily of preterm or sick neonates, characterized by mucosal or even deeper intestinal necrosis. It is the most common GI emergency among neonates. Symptoms and signs include feeding intolerance, lethargy, temperature instability, ileus, bloating, bilious emesis, hematochezia, reducing substances in the stool, apnea, and sometimes signs of sepsis. Diagnosis is clinical and is confirmed by imaging studies. Treatment is primarily supportive and includes nasogastric suction, parenteral fluids, TPN, and antibiotics. There currently exists no effective therapeutic or prophylactic approach for NEC and its associated systemic inflammation in premature infants. Treatment for a baby that may have necrotizing entercolis include halting regular feedings; relieving gas in the bowel by inserting a tube in the stomach; giving intravenous fluids and antibiotic medicines; monitoring the condition with abdominal x-rays, blood tests, and measurement of blood gases. The infant will need surgery if there is perforation of the intestines or inflammation of the abdominal wall (peritonitis). Surgery is used to remove dead bowel tissue, and may require a colostomy or ileostomy, and may require several weeks before the bowel can be reconnected.

WO 2015/038493 is entitled "Synthesis of novel asymmetric bow-tie PAMAM dendrimer-based conjugates for tumor-targeting drug delivery" and was published on 19 March 2015.

Teo Ian et al., Embo Molecular Medicine, (201209), vol. 4, no. 9, ISSN 1757-4684, pages 866 - 881 is entitled "Preventing acute gut wall damage in infectious diarrhoeas with glycosylated dendrimers".

US 2003/180250 is entitled "Compositions and complexes containing a macromolecular compound as potential anti-inflammatory agents" and was published on 25 September 2003.

Islam Dilara et al., Immunology (201602), vol. 147, no. 2, ISSN 1365-2567, pages 178 - 189 is entitled "Controlling the cytokine storm in severe bacterial diarrhoea with an oral Toll-like receptor 4 antagonist".

Navath Raghavendra S et al., Bioconjugate Chemistry, (200812), vol. 19, no. 12, ISSN 1520-4812, pages 2446 - 2455 is entitled "Dendrimer-drug conjugates for tailored intracellular drug release based on glutathione levels".

Mishra Manoj K et al., ACS Nano, (20140325), vol. 8, no. 3, ISSN 1936-086X, pages 2134 - 2147 is entitled "Dendrimer brain uptake and targeted therapy for brain injury in a large animal model of hypothermic circulatory arrest".

Yiyun C et al., European Journal of Medicinal Chemistry, Editions Scientifique Elsevier, Paris, FR, vol. 40, no. 12, ISSN 0223-5234, (20051201), pages 1390 - 1393, (20051201) is entitled "Polyamidoamine dendrimers used as solubility enhancers of ketoprofen".

S. Sadekar et al., Advanced Drug Delivery Reviews, (20120501), vol. 64, no. 6, ISSN 0169-409X, pages 571 - 588 is entitled "Transepithelial transport and toxicity of PAMAM dendrimers: Implications for oral drug delivery".

Fang Min et al., The Journal of Physical Chenmistry. B, (20120315), vol. 116, no. 10, ISSN 1520-5207, pages 3075 - 3082 is entitled "Host-guest chemistry of dendrimer-drug complexes: 7. Formation of stable inclusions between acetylated dendrimers and drugs bearing multiple charges".

It is therefore an object of the present invention to provide improved delivery to the gastrointestinal tract.

It is a further object of the invention to provide means of treating inflammatory disorders of the gastrointestinal tract, especially enterocolitis.

### SUMMARY OF THE INVENTION

The invention is set out in the appended set of claims. A pharmaceutical composition including dendrimers delivering therapeutic, prophylactic and/or diagnostic agents can be administered orally to reach target cells in the gastrointestinal tract, for treatment of an inflammatory gastrointestinal disorder, as well as the brain. As demonstrated by the example, the formulation was effective in treating necrotizing enterocolitis (NEC), a severe inflammatory condition that affects the gastrointestinal tract of premature infants, and is characterized by the sudden development of intestinal necrosis followed by systemic sepsis and death in over 40% of cases. The development of NEC requires the activation of the bacterial receptor toll like receptor 4 (TLR4) on the intestinal epithelium, which leads to activation of the major immune cells of the brain, the microglia. Microglia activation initiates an inflammatory cascade which results in the loss of myelin in the prefrontal cortex, and the development of cognitive impairment in mice. Importantly, the structural and inflammatory changes that are observed in mice closely resemble the changes observed in humans who develop this disease, as revealed by immunostaining of sections of human brains obtained at autopsy.

Oral administration of poly(amidoamine) dendrimers according to the claimed invention target inflammation in the gastrointestinal (GI) tract as well as the central nervous system (CNS) and deliver drugs that are capable of producing functional improvements. Oral administration of the dendrimer leads to significant concentration of the dendrimer in the injured areas of the gut and the brain in mice with NEC, with further selective localization in the inflammatory cells. Strikingly, oral administration of an anti-inflammatory agent (N-acetyl cysteine) using dendrimer results in dramatic improvement of the brain injury and gut injury in animals with NEC. This selective localization of the dendrimer in the injured gut and brain demonstrates that the oral dendrimer formulation should be useful for non-surgical treatment of NEC with preservation of the gut along with treatment of the associated systemic inflammation including neuroinflammation resulting in brain injury. In addition, by selectively localizing the fluorophore-tagged dendrimer in the inflammatory cells in the gut and brain, this technology may also represent a diagnostic tool for sensitive detection of NEC.

In the preferred embodiment, the dendrimer is used for delivery to the gut and the brain of anti-inflammatory agents and TLR4 inhibitors conjugated to the dendrimer. This provides a non-surgical option of preserving the gut and prevention/treatment of associated brain injury in premature neonates with NEC, to prevent or alleviate injury of both the GI tract and the brain. By co-administering a diagnostic agent, the dendrimers can also be used to provide non-invasive, real time detection of inflammation and injury in the gut and brain in NEC. The selective localization of dendrimer nanodevices in cells associated with inflammation provides an approach for the non-invasive, real time detection of inflammation and injury in the gut and brain in NEC. A preferred diagnostic is a fluorophore approved for human use such as indocyanine green for non-invasive detection.

A preferred formulation includes PAMAM dendrimer (4-6 generation) having N-acetyl cysteine bound thereto in combination with a TLR4 inhibitor. Preferred TLR4 inhibitors including small molecule inhibitors which inhibit TLR4 signaling, in particular C34 {Neal, 2013 PLoS One. 2013; 8(6): e65779. } .

### DETAILED DESCRIPTION OF THE INVENTION

### I. Definitions

The term "therapeutic agent" refers to an agent that can be administered to prevent or treat one or more symptoms of a disease or disorder. Examples include, but are not limited to, a nucleic acid, a nucleic acid analog, a small molecule, a peptidomimetic, a protein, peptide, carbohydrate or sugar, lipid, or surfactant, or a combination thereof.

The term "treating" refers to preventing or alleviating one or more symptoms of a disease, disorder or condition. Treating the disease or condition includes ameliorating at least one symptom of the particular disease or condition, even if the underlying pathophysiology is not affected, such as treating the pain of a subject by administration of an analgesic agent even though such agent does not treat the cause of the pain.

The phrase "pharmaceutically acceptable" refers to compositions, polymers and other materials and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. The phrase "pharmaceutically acceptable carrier" refers to pharmaceutically acceptable materials, compositions or vehicles, such as a liquid or solid filler, diluent, solvent or encapsulating material involved in carrying or transporting any subject composition, from one organ, or portion of the body, to another organ, or portion of the body. Each carrier must be "acceptable" in the sense of being compatible with the other ingredients of a subject composition and not injurious to the patient.

The phrase "therapeutically effective amount" refers to an amount of the therapeutic agent that produces some desired effect at a reasonable benefit/risk ratio applicable to any medical treatment. The effective amount may vary depending on such factors as the disease or condition being treated, the particular targeted constructs being administered, the size of the subject, or the severity of the disease or condition. One of ordinary skill in the art may empirically determine the effective amount of a particular compound without necessitating undue experimentation.

### II. Formulation

### A. Dendrimers

The term "dendrimer" as used herein includes, but is not limited to, a molecular architecture with an interior core, interior layers (or "generations") of repeating units regularly attached to this initiator core, and an exterior surface of terminal groups attached to the outermost generation. Examples of dendrimers include, but are not limited to, PAMAM, polyester, polylysine, and PPI. The PAMAM dendrimers have hydroxyl terminations and can be any generation of dendrimers including, but not limited to, generation 1 PAMAM dendrimers, generation 2 PAMAM dendrimers, generation 3 PAMAM dendrimers, generation 4 PAMAM dendrimers, generation 5 PAMAM dendrimers, generation 6 PAMAM dendrimers, generation 7 PAMAM dendrimers, generation 8 PAMAM dendrimers, generation 9 PAMAM dendrimers, or generation 10 PAMAM dendrimers. The invention concerns a dendrimer complex for use as claimed in the appended claims, wherein the dendrimer complex consists of a poly(amidoamine) (PAMAM) hydroxyl-terminated dendrimer having one or more anti-inflammatory agents conjugated to the dendrimer. Dendrimers suitable for use with include, but are not limited to, polyamidoamine (PAMAM), polypropylamine (POPAM), polyethylenimine, polylysine, polyester, iptycene, aliphatic poly(ether), and/or aromatic polyether dendrimers. The multiarm PEG polymer includes a polyethylene glycol having at least two branches bearing sulfhydryl or thiopyridine terminal groups; however, PEG polymers bearing other terminal groups such as succinimidyl or maleimide terminations can be used. The PEG polymers in the molecular weight 10 kDa to 80 kDa can be used.

As used herein, the term "PAMAM dendrimer" means poly(amidoamine) dendrimer, which may contain different cores, with amidoamine building blocks. The method for making them is known to those of skill in the art and generally, involves a two-step iterative reaction sequence that produces concentric shells (generations) of dendritic β-alanine units around a central initiator core. This PAMAM core-shell architecture grows linearly in diameter as a function of added shells (generations). Meanwhile, the surface groups amplify exponentially at each generation according to dendritic-branching mathematics. They are available in generations G0 - 10 with 5 different core types and 10 functional surface groups. The dendrimer-branched polymer consists of polyamidoamine (PAMAM) polypeptide dendrimers.

In accordance with some embodiments, the PAMAM dendrimers used can be generation 4 dendrimers, or more, with hydroxyl groups attached to their functional surface groups. The multiarm PEG polymer comprises polyethylene glycol having 2 and more branches bearing sulfhydryl or thiopyridine terminal groups; however, PEG polymers bearing other terminal groups such as succinimidyl or maleimide terminations can be used. The PEG polymers in the molecular weight 10 kDa to 80 kDa can be used.

In some embodiments, the dendrimers are in nanoparticle form and are described in detail in international patent publication No. WO2009/046446.

### Preparation of PAMAM-NAC

Below is a synthetic scheme for conjugating *N*-acetylcysteine to an amine-terminated fourth generation PAMAM dendrimer (PAMAM-NH₂), using N-succinimidyl 3-(2-pyridyldithio)propionate (SPDP) as a linker.

Synthesis of *N*-succinimidyl 3-(2-pyridyldithio)propionate (SPDP) is performed by a two-step procedure, Scheme 1. First, 3-mercaptopropionic acid is reacted by thiol-disulfide exchange with 2,2'-dipyridyl disulfide to give 2-carboxyethyl 2-pyridyl disulfide. To facilitate linking of amine-terminated dendrimers to SPDP, the succinimide group is reacted with 2-carboxyethyl 2-pyridyl disulfide to obtain *N*-succinimidyl 3-(2-pyridyldithio)propionate, by esterification with N-hydroxysuccinimide by using *N*,*N'*-dicyclohexylcarbodiimide and 4-dimethylaminopyridine.

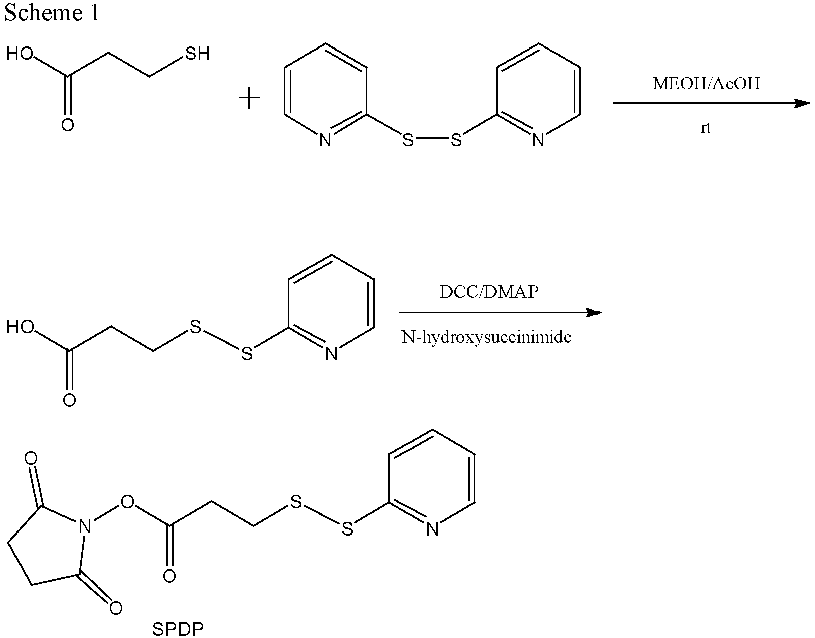

To introduce sulfhydryl-reactive groups, PAMAM-NH₂ dendrimers are reacted with the heterobifunctional cross-linker SPDP, Scheme 2. The *N-*succinimidyl activated ester of SPDP couples to the terminal primary amines to yield amide-linked 2-pyridyldithiopropanoyl (PDP) groups, Scheme 2. After the reaction with SPDP, PAMAM-NH-PDP can be analyzed using RP-HPLC to determine the extent to which SPDP has reacted with the dendrimers.

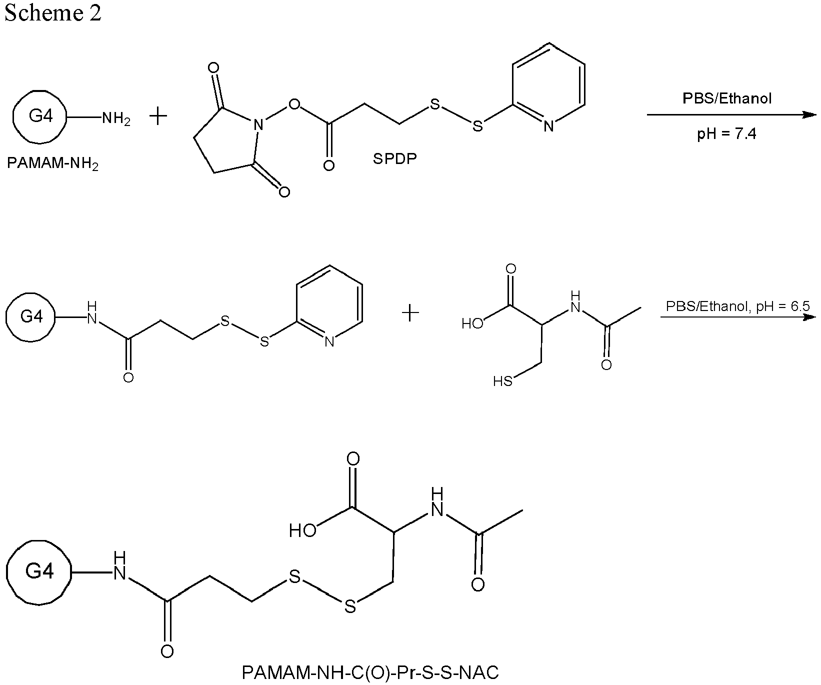

In another embodiment, the synthetic routes described in Scheme 4, below, can be used in order to synthesize D-NAC up to the pyridyldithio (PDP)-functionalized dendrimer **3.** Compound **3** is then reacted with NAC in DMSO, overnight at room temperature to obtain D-NAC **5.**

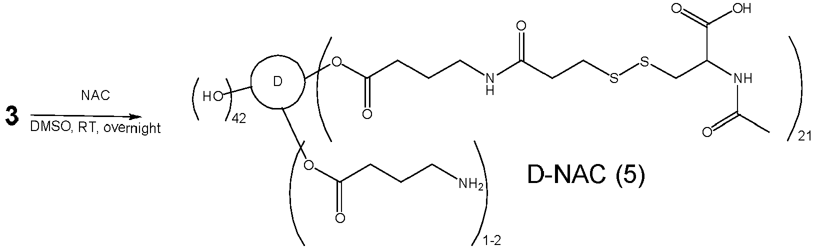

### Preparation of Dendrimer-PEG-valproic acid conjugate (D-VPA)

Initially, valproic acid is functionalized with a thiol-reactive group. A short PEG-SH having three repeating units of (CH₂)₂O- is reacted with valproic acid using DCC as coupling reagent as shown in Scheme 3. The crude PEG-VPA obtained is purified by column chromatography and characterized by proton NMR. In the NMR spectrum, there was a down-shift of the peak of CH₂ protons neighboring to OH group of PEG to 4.25 ppm from 3.65 ppm that confirmed the formation of PEG-VPA. Although the thiol group also may be susceptible to reacting with acid functionality, the NMR spectra did not indicate any downward shift of the peak belonging to CH₂ protons adjacent to thiol group of PEG. This suggests that the thiol group is free to react with the thiol-reactive functionalized dendrimer.

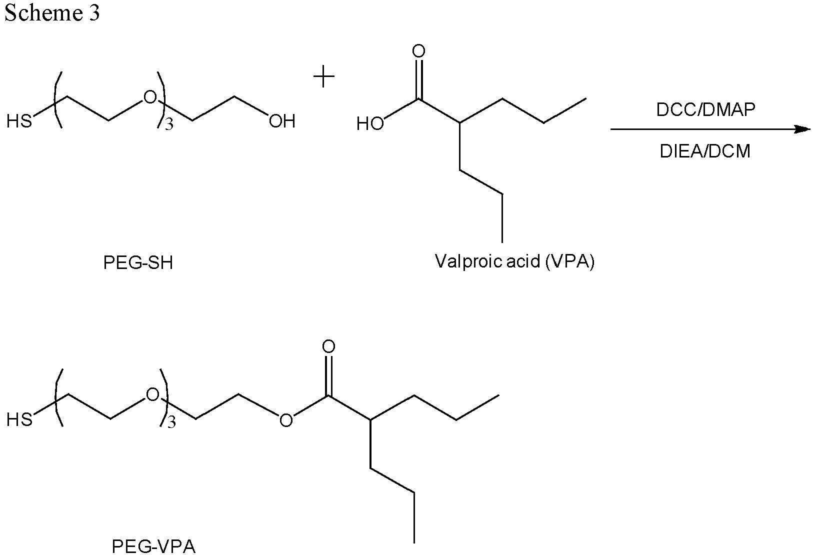

To conjugate PEG-VPA to the PAMAM-OH, a disulfide bond is introduced between the dendrimer and valproic acid, Scheme 4. First the dendrimer is converted to a bifunctional dendrimer **1** by reacting the dendrimer with fluorenylmethyloxycarbonyl (Fmoc) protected γ-aminobutyric acid (GABA). Conjugation of PEG-VPA to the bifunctional dendrimer involved a two-step process: the first step is the reaction of amine-functionalized bifunctional dendrimer **1** with *N*-succinimidyl-3-(2-pyridyldithio)-propionate (SPDP), and the second step involves conjugating the thiol-functionalized valproic acid. SPDP is reacted with the intermediate **2** in the presence of *N*,*N*-diisopropylethylamine (DIEA) to obtain pyridyldithio (PDP)-functionalized dendrimer **3.**

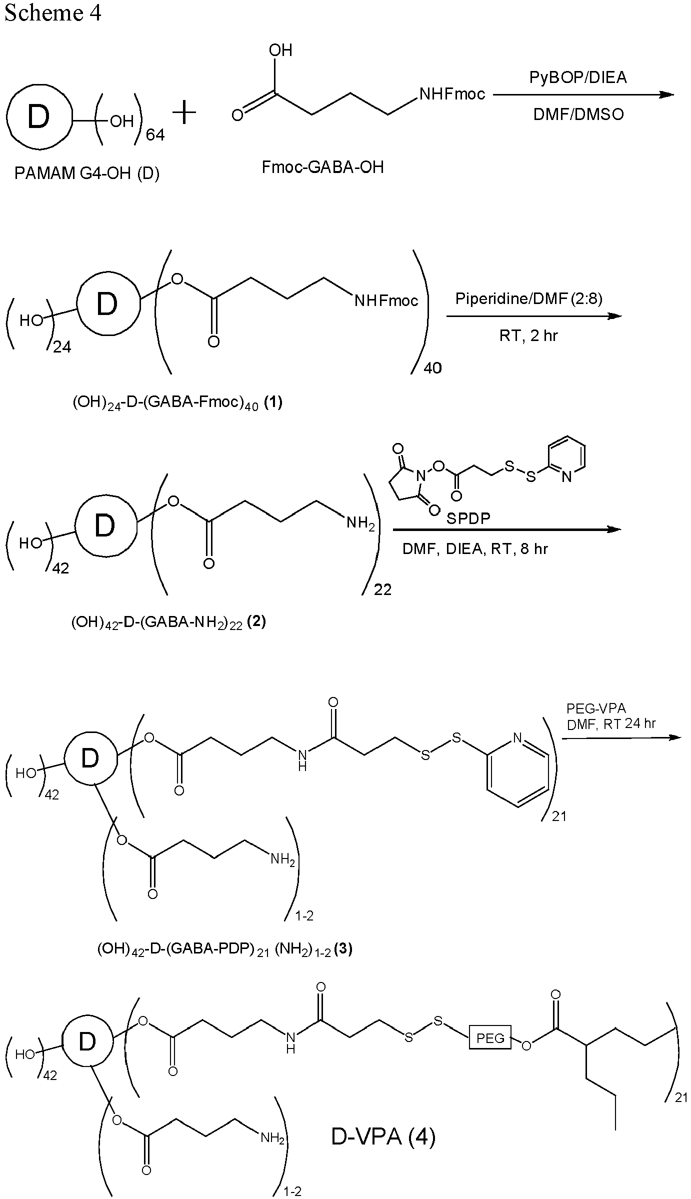

Even though this is an *in situ* reaction process, the structure was established by ¹H NMR. In the spectrum, new peaks between 6.7 and 7.6 ppm for aromatic protons of pyridyl groups confirmed the formation of the product. The number of pyridyl groups and number of GABA linkers were verified to be the same, which indicates that most of the amine groups reacted with the SPDP. Since this is a key step for the conjugation of the drug to the dendrimer, the use of mole equivalents of SPDP per amine group and time required for the reaction was validated. Finally, the PEG-VPA is reacted with the PDP-functionalized dendrimer *in situ* to get dendrimer-PEG-valproic acid (D-VPA). The formation of the final conjugate and loading of VPA were confirmed by ¹H NMR, and the purity of the conjugate was evaluated by reverse-phase HPLC. In the NMR spectrum, multiplets between 0.85 and 1.67 ppm for aliphatic protons of VPA, multiplets between 3.53 and 3.66 ppm for CH₂ protons of PEG, and absence of pyridyl aromatic protons confirmed the conjugate formation. The loading of the VPA is ~21 molecules, estimated using a proton integration method, which suggests that 1-2 amine groups are left unreacted. In the HPLC chart, the elution time of D-VPA (17.2 min) is different from that for G4-OH (9.5 min), confirming that the conjugate is pure, with no measurable traces of VPA (23.4 min) and PEG-VPA (39.2 min).The percentage of VPA loading to the dendrimer is ~12% w/w and validates the method for making gram quantities in three different batches.

### B. Coupling Agents and Spacers

Dendrimer complexes can be formed of therapeutically active agents or compounds (hereinafter "agent") conjugated or attached to a dendrimer or multiarm PEG. Dendrimer complexes used in the invention are formed of one or more anti-inflammatory agents conjugated to a poly(amidoamine) (PAMAM) hydroxyl-terminated dendrimer. The attachment can occur via an appropriate spacer that provides a disulfide bridge between the agent and the dendrimer. The dendrimer complexes are capable of rapid release of the agent in vivo by thiol exchange reactions, under the reduced conditions found in body.

The term "spacers" as used herein is intended to include compositions used for linking a therapeutically active agent to the dendrimer. The spacer can be either a single chemical entity or two or more chemical entities linked together to bridge the polymer and the therapeutic agent or imaging agent. The spacers can include any small chemical entity, peptide or polymers having sulfhydryl, thiopyridine, succinimidyl, maleimide, vinylsulfone, and carbonate terminations.

The spacer can be chosen from among a class of compounds terminating in sulfhydryl, thiopyridine, succinimidyl, maleimide, vinylsulfone and carbonate group. The spacer can comprise thiopyridine terminated compounds such as dithiodipyridine, N-Succinimidyl 3-(2-pyridyldithio)-propionate (SPDP), Succinimidyl 6-(3-[2-pyridyldithio]-propionamido)hexanoate LC-SPDP or Sulfo-LC-SPDP. The spacer can also include peptides wherein the peptides are linear or cyclic essentially having sulfhydryl groups such as glutathione, homocysteine, cysteine and its derivatives, arg-gly-asp-cys (RGDC), cyclo(Arg-Gly-Asp-d-Phe-Cys) (c(RGDfC)), cyclo(Arg-Gly-Asp-D-Tyr-Cys), cyclo(Arg-Ala-Asp-d-Tyr-Cys). The spacer can be a mercapto acid derivative such as 3 mercapto propionic acid, mercapto acetic acid, 4 mercapto butyric acid, thiolan-2-one, 6 mercaptohexanoic acid, 5 mercapto valeric acid and other mercapto derivatives such as 2 mercaptoethanol and 2 mercaptoethylamine. The spacer can be thiosalicylic acid and its derivatives, (4-succinimidyloxycarbonyl-methyl-alpha-2-pyridylthio)toluene, (3-[2-pyridithio]propionyl hydrazide, The spacer can have maleimide terminations wherein the spacer comprises polymer or small chemical entity such as bis-maleimido diethylene glycol and bis-maleimido triethylene glycol, Bis-Maleimidoethane, bismaleimidohexane. The spacer can comprise vinylsulfone such as 1,6-Hexane-bis-vinylsulfone. The spacer can comprise thioglycosides such as thioglucose. The spacer can be reduced proteins such as bovine serum albumin and human serum albumin, any thiol terminated compound capable of forming disulfide bonds The spacer can include polyethylene glycol having maleimide, succinimidyl and thiol terminations.

### C. Therapeutic, Prophylactic and Diagnostic Agents

The term "dendrimer complexes" as used herein refers to the combination of a poly(amidoamine) (PAMAM) hydroxyl-terminated dendrimer with an anti-inflammatory agent. These dendrimer complexes include an agent that is attached or conjugated to PAMAM dendrimers, which are capable of preferentially releasing the drug intracellularly under the reduced conditions found *in vivo.* The dendrimer complex, when administered by i.v. injection, can preferentially cross the blood brain barrier (BBB) only under diseased condition and not under normal conditions.

The anti-inflammatory agent can be either covalently attached or intra-molecularly dispersed or encapsulated. The dendrimer is preferably a PAMAM dendrimer up to generation 10, having hydroxyl terminations. The dendrimer is linked to the anti-inflammatory agent via a spacer ending in disulfide, ester or amide bonds.

Representative anti-inflammatory therapeutic, prophylactic or diagnostic agents can be peptides, proteins, carbohydrates, nucleotides or oligonucleotides, small molecules, or combinations thereof.

A preferred antiinflammatory is an antioxidant drug including N-acetylcysteine. Preferred NSAIDS include mefenamic acid, aspirin, Diflunisal, Salsalate, Ibuprofen, Naproxen, Fenoprofen, Ketoprofen, Deacketoprofen, Flurbiprofen, Oxaprozin, Loxoprofen, Indomethacin, Sulindac, Etodolac, Ketorolac, Diclofenac, Nabumetone, Piroxicam, Meloxicam, Tenoxicam, Droxicam, Lornoxicam, Isoxicam, Meclofenamic acid, Flufenamic acid, Tolfenamic acid, elecoxib, Rofecoxib, Valdecoxib, Parecoxib, Lumiracoxib, Etoricoxib, Firocoxib, Sulphonanilides, Nimesulide, Niflumic acid, and Licofelone.

Representative small molecules include steroids such as methyl prednisone, dexamethasone, non-steroidal anti-inflammatory agents, including COX-2 inhibitors, corticosteroid anti-inflammatory agents, gold compound anti-inflammatory agents, immunosuppressive, anti-inflammatory and anti-angiogenic agents, anti-excitotoxic agents such as valproic acid, D-aminophosphonovalerate, D-aminophosphonoheptanoate, inhibitors of glutamate formation/release, such as baclofen, NMDA receptor antagonists, salicylate anti-inflammatory agents, ranibizumab, anti-VEGF agents, including aflibercept, and rapamycin. Other anti-inflammatory drugs include nonsteroidal drug such as indomethacin, aspirin, acetaminophen, diclofenac sodium and ibuprofen. The corticosteroids can be fluocinolone acetonide and methylprednisolone. The peptide drug can be streptidokinase.

Many inflammatory diseases may be linked to pathologically elevated signaling via the receptor for lipopolysaccharide (LPS), toll-like receptor 4 (TLR4). There has thus been great interest in the discovery of TLR4 inhibitors as potential anti-inflammatory agents. Recently, the structure of TLR4 bound to the inhibitor E5564 was solved, enabling design and synthesis of new TLR4 inhibitors that target the E5564-binding domain. These are described in U.S. Patent No. 8,889,101. As reported by Neal, et al., PLoS One. 2013; 8(6): e65779e, a similarity search algorithm used in conjunction with a limited screening approach of small molecule libraries identified compounds that bind to the E5564 site and inhibit TLR4. The lead compound, C34, is a 2-acetamidopyranoside (MW 389) with the formula C₁₇H₂₇NO₉, which inhibits TLR4 in enterocytes and macrophages *in vitro,* and reduces systemic inflammation in mouse models of endotoxemia and necrotizing enterocolitis. Molecular docking of C34 to the hydrophobic internal pocket of the TLR4 co-receptor MD-2 demonstrated a tight fit, embedding the pyran ring deep inside the pocket. Strikingly, C34 inhibited LPS signaling ex-vivo in human ileum that was resected from infants with necrotizing enterocolitis. These findings identify C34 and the β-anomeric cyclohexyl analog C35 as novel leads for small molecule TLR4 inhibitors that have potential therapeutic benefit for TLR4-mediated inflammatory diseases.

Wipf, et al., Tetrahedron Lett. 2015 56(23):3097-3100 ("Wipf"), describes analogues of C34. A copper(II)-mediated solvolysis of anomeric oxazolines and an acid-mediated conversion of β-glucosamine and β-galactosamine pentaacetates were used to generate analogs of C34 at the anomeric carbon and at C-4 of the pyranose ring. These compounds were evaluated for their influence on TLR4-mediated inflammatory signaling in cultured enterocytes and monocytes. Their efficacy was confirmed using a NF-kB-luciferase reporter mouse, thus establishing the first structure-activity relationship (SAR) study in this series and identifying the more efficacious isopropyl 2-acetamido-α-galactoside **17.** These data show that C34, its analogues, or other TLR4 inhibitors can be conjugated to the dendrimers for use in the oral formulations.

The dendrimer complex can also be used to deliver anti-excitotoxic and D-anti-glutamate agents. Preferred candidates are: MK801, Memantine, Ketamine, 1-MT.

Representative oligonucleotides include siRNAs, microRNAs, DNA, and RNA. The therapeutic agent can be a PAMAM dendrimer with amine or hydroxyl terminations.

The dendrimer complexes linked to a bioactive compound or therapeutically active agent can be used to perform several functions including targeting, localization at a diseased site, releasing the drug, and imaging purposes. The dendrimer complexes can be tagged with or without targeting moieties such that a disulfide bond between the dendrimer and the agent or imaging agent is formed via a spacer or linker molecule.

### D. Devices and Formulations

The dendrimers of the present invention are administered orally. The carriers or diluents used herein may be solid carriers or diluents for solid formulations, liquid carriers or diluents for liquid formulations, or mixtures thereof.

For liquid formulations, pharmaceutically acceptable carriers may be, for example, aqueous or non-aqueous solutions, suspensions, emulsions or oils. Examples of non-aqueous solvents are propylene glycol, polyethylene glycol, and injectable organic esters such as ethyl oleate. Aqueous carriers include, for example, water, alcoholic/aqueous solutions, cyclodextrins, emulsions or suspensions, including saline and buffered media.

Examples of oils are those of petroleum, animal, vegetable, or synthetic origin, for example, peanut oil, soybean oil, mineral oil, olive oil, sunflower oil, fish-liver oil, sesame oil, cottonseed oil, corn oil, olive, petrolatum, and mineral. Suitable fatty acids for use in parenteral formulations include, for example, oleic acid, stearic acid, and isostearic acid. Ethyl oleate and isopropyl myristate are examples of suitable fatty acid esters.

Vehicles include, for example, sodium chloride solution, Ringer's dextrose, dextrose and sodium chloride, lactated Ringer's and fixed oils. Formulations include, for example, aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. Vehicles can include, for example, fluid and nutrient replenishers, electrolyte replenishers such as those based on Ringer's dextrose. In general, water, saline, aqueous dextrose and related sugar solutions are preferred liquid carriers. These can also be formulated with proteins, fats, saccharides and other components of infant formulas.

### III. Methods of Treatment

### A. Disorders or Diseases to be Treated

The invention set out in the appended claim is directed to dendrimer complexes for use in treating an inflammatory gastrointestinal disorder characterised by inflammation in the gastrointestinal tract and/or central nervous system. The innate immune receptor toll-like receptor 4 (TLR4) has been recognized as the receptor on hematopoietic and non-hematopoietic cells for bacterial endotoxin (lipopolysaccharide, "LPS"), as well as for a variety of endogenous molecules that are released during inflammatory or infectious disorders. A number of diseases have been attributed to exaggerated TLR4 signaling, including both infectious and non-infectious processes. These include necrotizing enterocolitis (NEC), abdominal sepsis, pneumonia, arthritis, pancreatitis and atherosclerosis. In a preferred embodiment, the disease to be treated is NEC.

The dendrimer complex composition, including a dendrimer linked to one or more anti-inflammatory agents, can selectively target microglia and astrocytes, which play a key role in the pathogenesis of NEC. N-acetyl cysteine ("NAC") has been extensively investigated and studied. It is also investigated for neuro-inflammation associated in maternal fetal infections. However, NAC suffers from low bioavailability due to high plasma protein binding. The dendrimer complex compositions overcome the plasma protein binding without affecting the activity of NAC. G4 PAMAM-NAC can be ten to a hundred times more efficacious *in vivo* than the free drug NAC by single i.v. administration. The free drug NAC exhibits very high plasma protein binding resulting in reduced bioavailability. One of the major advantages of this dendrimer complex is that it enhances the bioavailability by restricting the unwanted drug plasma protein interactions and selectively results in rapid release of the drug intracellularly to exhibit the desired therapeutic action.

The high payload of the drug NAC in the G4 PAMAM-NAC requires very small quantities (10 mg) of the carrier, PAMAM dendrimer, thereby reducing the amounts administered daily. A decreased quantity of agent limits the side effects associated with the agent. Since the bioavailability of the agent remains high, the positive effects of the agent are not lowered despite the administration of smaller quantities of agent. The dendrimer complexes including the dendrimer-drug conjugates, restricts its biodistribution to tissues and organ and preferentially deliver the drug at the target site thereby reducing the undesired side effects.

Dendrimer complexes effectively transport across the BBB, and are therefore useful for targeted drug delivery in neurological, neurodevelopmental, and neurodegenerative disorders and brain injury. G4-PAMAM-S--S-NAC conjugates specifically target activated microglial cells and astrocytes in neuroinflammatory disorders:
The therapeutic efficacy of G4-PAMAM-S--S-NAC dendrimer conjugate was evaluated after two days of animal treatment with lipopolysaccharide (LPS) to induce white matter injury and hypomyelination in the developing rabbit brain (an animal model of Cerebral Palsy). NAC selectively delivered from the G4-PAMAM-S--S-NAC dendrimer complexes strongly suppressed pro-inflammatory cytokines (TNF-.alpha., IL-6 mRNA), inflammatory signaling factors, including NF.kappa.B and nitrotyrosine, and enhanced GSH level. The G4-PAMAM-S--S-NAC was found to be ten to a hundred times more efficacious compared with free NAC. This supports a conclusion that the G4-PAMAM-S--S-NAC traversed across the BBB. The targeted delivery of NAC from dendrimer complex to actived microglial cells improved the motor deficits and attenuated recovery from the LPS-induced brain injury in a neonatal rabbit model of cerebral palsy.

A significant reduction in proinflammatory cytokines (TNF-.alpha., IL-6 mRNA) was observed on administration of G4-PAMAM-S--S-NAC dendrimer complexes. The kits treated with NAC and G4-PAMAM-S--S-NAC showed a decrease in fetal inflammation response with improvement of motor deficits when compared to the kits that were treated with saline. The kits that were treated with G4-PAMAM-S--S-NAC conjugates had less behavioral changes and lower microglial activation in the brain when compared to the kits that received NAC alone due to the sustained delivery of NAC from G4-PAMAM-S--S-NAC conjugate. The results indicated that G4-PAMAM-S--S-NAC conjugates have a greater effect than NAC alone since it is preferentially taken up by activated macrophages and microglial cells, reducing the inflammatory and oxidative and nitrosative effects.

Treatment with G4-PAMAM-S--S-NAC dendrimer complexes reduced white matter injury and microglia activation. A significant reduction in dose of NAC was observed when administered as G4-PAMAM-S--S-NAC to elicit the similar response as that observed for free NAC. Both free NAC at concentration 100 mg/kg and G4-PAMAM-S--S-NAC at concentration 10 mg/kg, 10 mg elicit identical responses, demonstrating that on conjugating to dendrimer a reduction in dose is achieved. G4-PAMAM-S- -S-NAC at lower concentrations than free NAC shows significant protective effects against LPS-induced brain injuries, suppression of TNF-.alpha, and down-regulation of IL-6 activity. This activity of the dendrimer-NAC conjugates may be attributed to its ability to interfere with the early inflammatory responses by blocking or modifying the signal transduction factor NF-.kappa.B and nitrotyrosine, thereby modulating cellular activation. 6 and 8 arm PEG-NAC conjugates released 74% of NAC in the intracellular GSH concentration (2 and 10 mM), within 2 hours. At a concentration range of between 0.008-0.8 mM, the conjugates were nontoxic to the microglial cells. At an equimolar concentration of NAC (0.5 mM) the 6-arm-PEG-S--S-NAC and 8-arm-PEG-S--S-NAC were more efficient in inhibition of GSH depletion than the free NAC. Both 6 and 8-arm-PEG-S--S-NAC conjugates, each at 0.5 mM and 5 Mm concentration showed significant inhibition in ROS production when compared to free NAC at equimolar concentrations. The studies demonstrate that the conjugates are superior in inhibition of the NO production as compared to the free NAC. At the highest concentration (5 mM), the free drug reduced the H₂O₂ levels and nitrite levels by 30-40%, whereas the conjugates reduced the H₂O₂ and nitrite levels by more than 70%. This shows that the conjugates are able to traffic the drug inside the cells, and release the drug in the free form and are significantly more efficacious than the free drug. At 5 mM concentration 6-arm-PEG-S--S-NAC conjugate (1) showed significant inhibition (70%) of TNF-.alpha, production when compared to equivalent concentration of NAC (Pb0.05). 8-arm-PEG-S- -S-NAC conjugate (3) showed significant inhibition of TNF-.alpha. production (70%) at 5 mM when compared to equivalent concentration of NAC (Pb0.05 and Pb0.01). PEGylated NAC is a dendrimer complex with utility for the pharmaceutical industry, as PEGs are approved for human use and this device addresses limitations of NAC and provides greater efficacy.

### B. Dosages

Typically, an attending physician will decide the dosage of the composition with which to treat each individual subject, taking into consideration a variety of factors, such as age, body weight, general health, diet, sex, compound to be administered, route of administration, and the severity of the condition being treated. The dose of the compositions can be about 0.0001 to about 1000 mg/kg body weight of the subject being treated, from about 0.01 to about 100 mg/kg body weight, from about 0.1 mg/kg to about 10 mg/kg, and from about 0.5 mg to about 5 mg/kg body weight

In general the timing and frequency of administration will be adjusted to balance the efficacy of a given treatment or diagnostic schedule with the side-effects of the given delivery system. Exemplary dosing frequencies include continuous infusion, single and multiple administrations such as hourly, daily, weekly, monthly or yearly dosing.

It will be understood by those of ordinary skill that a dosing regimen used in the inventive methods can be any length of time sufficient to treat the disorder in the subject. The term "chronic" as used herein, means that the length of time of the dosage regimen can be hours, days, weeks, months, or possibly years.

The dendrimer complexes can be administered in combination with one or more additional therapeutically active agents, which are known to be capable of treating conditions or diseases discussed above.

The present invention will be further understood by reference to the following examples

### Example 1: Treatment of Necrotizing Enterocolitis

The development of NEC requires the activation of the bacterial receptor toll like receptor 4 (TLR4) on the intestinal epithelium, as mice lacking TLR4 are protected from NEC, while humans with NEC exhibit increased TLR4 activation in the gut. Activation of TLR4 on the intestinal epithelium leads to activation of microglia, resulting in the loss of myelin in the prefrontal cortex, and the development of cognitive impairment in mice in a manner that closely resembles the disease observed in humans.

Poly(amidoamine) dendrimers target inflammation in the CNS and deliver drugs to produce functional improvements. As demonstrated by the following example, oral administration of the dendrimer leads to significant accumulation of the dendrimer in the injured areas of the gut and the brain in mice with NEC, with further selective localization in the inflammatory cells. Strikingly, oral administration of an anti-inflammatory agent (N-acetyl cysteine) using dendrimer results in dramatic improvement of the brain injury and gut injury in animals with NEC. This selective localization of the dendrimer in the injured gut and brain has implications for non-surgical treatment of NEC with preservation of the gut along with treatment of the associated systemic inflammation including neuroinflammation resulting in brain injury. The selective localization of the fluorophore-tagged dendrimer in the inflammatory cells in the gut can also be used as a diagnostic tool for sensitive detection of NEC.

### Materials and Methods

**Dendrimers.** Detailed materials and methods used in the experiments below, including protocols for making the dendrimers-Cy5 and dendrimers-drug conjugates, have been described by Kannan S et al Sci. Transl. Med., 4:130ra46 (2012) and in U.S. Patent No. 8,889,101.

Conjugation of dendrimer conjugates. The conjugation of dendrimers to Cy5 was done using previously reported methods (Kannan et al., Science Trans. Med (April, 2012). For drug experiments, dendrimers were conjugated to N-acetyl-cysteine and administered at doses ranging from 2-20 mg/kg at differing time points.

Statistical analysis. The data was analyzed for the reproducibility using Student's t-test to determine the significance between two groups. A p-value equal to or less than 0.05 was considered significant.

**Animal model of NEC.** Neonatal mice (postnatal day 6 - 11) were exposed to a well-established model of necrotizing enterocolitis (NEC). Briefly, pups were fed formula supplemented with bacteria isolated from human NEC via gavage five times/day and submitted to hypoxia (5%O₂, 95% N₂) for 10 min in a hypoxic chamber twice daily for 4 days.

Two experimental protocols were employed to determine the acute versus long-term effects of NEC on the brain. To determine acute effects, brain samples were harvested immediately after the completion of the NEC protocol (postnatal day 11) followed by immunohistochemical evaluation of myelination and microglial activation. Behavioral implications of NEC exposure were determined 3 weeks after the completion of NEC protocol (postnatal day 32 - 46) using a Morris water maze and novel object recognition tests.

**Dendrimer-NAC therapies.** N-acetyl-cysteine conjugated dendrimers (D-NAC) delivered through oral gavage at a clinically relevant dose of 18mg/kg on a NAC basis (100 mg/kg on a D-NAC conjugate basis), on days 2 and 3 of the NEC protocol (*i.e*. postnatal days 8 and 9).

For imaging, the Cy5-labeled dendrimers (D-Cy5) were delivered through oral gavage at 100mg/kg on day 3 of NEC protocol, with few hours delay after the last dose of D-NAC.

High Performance Liquid Chromatography (HPLC) analysis. The purity of the dendrimer-Cy5 conjugates (D-Cy5) were analyzed using a Waters HPLC instrument (Waters Corporation, Milford, Massachusetts) equipped with Waters In-line degasser, binary pump, photodiode array (PDA) detector, multi fluorescence λ detector and auto sampler (maintained at 4°C) interfaced with Empower software. The HPLC chromatogram was monitored simultaneously for absorbance at 210 nm for dendrimer and 650nm for Cy5 using Waters 2998 PDA detector and fluorescence with excitation at 645 nm and emission at 662 nm using Waters 2475 fluorescence detector. The water/acetonitrile (0.1% w/w TFA) was freshly prepared, filtered, degassed, and used as a mobile phase. TSK-Gel ODS-80 Ts (250 X 4.6 mm, 25 cm length with 5 µm particle size) connected to TSK-Gel guard column was used. A gradient flow was used with initial condition being 90:10 (H2O/ACN) and then gradually increasing the acetonitrile concentration to 10:90 (H2O/ACN) in 30 min and returning to original initial condition 90:10 (H2O/ACN) in 60 min with flow rate of 1 ml/min.

Immunohistochemistry and confocal microscopy. Brian slices were fixed in 2% paraformaldehyde (PFA) in PBS. The brains were frozen in 20% sucrose with optimum cutting temperature compound (OCT) (Sakura Finetek USA Inc., Torrance, CA) in a 1:2 ratio respectfully using dry ice in isopentane. Cryoblocks are stored at -80 °C until sectioned. Eight µm sections were cut from frozen blocks using a cryostat. Sections were incubated in rabbit anti-Ionised Calcium Binding Adapter 1 molecule (Iba-1) (Wako chemicals, USA), which is a microglia cell marker, and a goat anti-rabbit-Cy3 secondary antibody applied. Sections were analysed on a Zeiss 510 confocal microscope. Excitation and emission wavelengths and laser settings were identical to analyze all tissue in IV injected animals. Z-stacks of sections were taken and collapsed to give an image through the depth of the whole section.

### Results

Pups exposed to NEC display a significant cognitive deficit, evidenced by impaired working memory and spatial learning in Morris water Maze and novel object recognition behavioral tests. These findings are correlated with histopathological evaluations of brain myelination and microglial activation. Animals exposed to NEC have a deficient myelination pattern when compared to control animals, which is particularly evident by the decreased expression of myelin basic protein (MBP) in the midbrain and corpus callosum. Furthermore, NEC treated animals display increased microglial activation at the level of the corpus callosum, hippocampus and midbrain compared to controls.

Orally-administered dendrimers are taken up readily by the gut, and exhibited pathology-dependent biodistribution. The dendrimer was mostly seen to accumulate in the cerebral cortex, especially the parietal cortex and the motor cortex.. D-Cy5 were also seen to accumulate at thalamus, thalamic nuclei.

Administration of D-NAC during NEC leads to a protective effect on the brain as evidenced by a reduced D-Cy5 uptake (indicative of a lower level of inflammation, reduced of microglial activation, and normal myelination pattern, compared to untreated NEC mice. The protective effects of D-NAC were observed despite the fact that animals display clear evidence of necrotizing enterocolitis by histopathological and qRT-PCR evaluation.

In all mice group dosed with Cy5-labeled dendrimers (D-Cy5), the dendrimers accumulated in the gut, taken up by the epithelial cells in the intestine villus, indicating D-Cy5 was absorbed in the GI track and partitioned in the systemic circulation.

In the formula fed (NEC) mice, Cy5 labeled dendrimers (D-Cy5) were mostly seen to accumulate in the cerebral cortex, especially the parietal cortex and the motor cortex (Figure 2). D-Cy5 were also seen to accumulate at thalamus, thalamic nuclei.

To further identify the cellular co-localization of D-Cy5 at the region of interests, the somatosensory cortex at the parietal region was examined under higher magnification. At the somatosensory cortex, most of the D-Cy5 localization seem to be in the layer 2-5 of the cortex, and forms a pattern of cellular uptake.

At thalamus and thalamic nuclei, D-Cy5 clearly showed cellular co-localization, with dominate cell uptake by unlabeled cells, and some uptake by activated microglia/macrophages.

In addition, D-NAC treatment significantly decreased the NEC associated microglial activation, D-Cy5 accumulation in the periventricular region and greatly enhanced the myelination in the brains of formula fed mice. D-NAC treatment decreased the D-Cy5 accumulation in the periventricular region of the brain in the formula fed mice. In breast fed mice (healthy positive controls, D-Cy5 were only seen at the choroid plexus. In formula fed mice (NEC negative control, D-Cy5 accumulated at the periventricular region with a great amount and formed a scattered pattern, presumably due to cellular uptake. After D-NAC treatment to the formula fed mice (NEC dendrimer-NAC treated), the D-Cy5 accumulation decreased significantly around the periventricular region, with only minimal D-Cy5 observed in the outlayer of ventrical. Since the dendrimer uptake in brain has been shown to be proportional to the extent of inflammation, this indicates that oral D-NAC treatment reduced neuroinflammation.

D-NAC treatment decreased the NEC associated microglial activation. In breast fed and formula fed + D-NAC treatment mice, microglial cells had similar population, with small cell bodies, suggestion microglial cells were in 'resting state'. In contrast, in the formula fed NEC mice (control), microglia population increased significantly, with enlarged cell bodies, suggesting microglial activation.

D-NAC treatment protected against the myelination defect associated with NEC in the brains of formula fed mice. The breast fed (healthy control) and formula fed + D-NAC treatment (NEC, treated with D-NAC) mice had a higher extent of myelination in the brain than the only formula fed mice (NEC untreated), indicating reduction of inflammation in the formula fed + D-NAC treatment group benefited the neurological repair.

Similar results have been obtained using a piglet model, described by Good, et al. Am J Physiol Gastrointest Liver Physiol. 2014 306(11):G1021-32. doi: 10.1152/ajpgi.00452.2013. Epub 2014 Apr 17.

## Claims

1. A dendrimer complex for use in a method for treating an inflammatory gastrointestinal disorder, the method comprising orally administering to a subject a dendrimer complex consisting of a poly(amidoamine) (PAMAM) hydroxyl-terminated dendrimer having one or more anti-inflammatory agents conjugated to the dendrimer, wherein the dendrimer complex crosses the blood brain barrier, wherein the use is in a subject that has inflammation in the gastrointestinal tract and/or a disorder with central nervous system complications.

2. The dendrimer complex for use according to claim 1, wherein the PAMAM dendrimer is a G3, G4, G5, or G6 PAMAM dendrimer.

3. The dendrimer complex for use according to any one of claims 1-2, wherein the PAMAM dendrimer is linked to the one or more anti-inflammatory agents via a disulfide bond.

4. The dendrimer complex for use according to any one of claims 1-3, wherein the PAMAM dendrimer is linked to the one or more anti-inflammatory agents via one or more spacer compounds selected from the group consisting of N-succinimidyl 3-(2-pyridyldithio)propionate (SPDP), Glutathione (GSH), Gamma-aminobutyric acid (GABA), and combinations thereof.

5. The dendrimer complex for use according to any one of claims 1-4, wherein at least one of the one or more anti-inflammatory agents is N-acetyl cysteine.

6. The dendrimer complex for use according to any one of claims 1-4, wherein at least one of the one or more anti-inflammatory agents is an inhibitor of toll like receptor 4.

7. The dendrimer complex for use according to any one of claims 1-6, wherein the disorder to be treated is necrotizing enterocolitis (NEC).

8. The dendrimer complex for use according to any one of claims 1-6, wherein the disorder to be treated is selected from the group consisting of abdominal sepsis and pancreatitis.

9. The dendrimer complex for use according to any one of claims 1-6, wherein the subject has inflammation in the gastrointestinal tract and a disorder with central nervous system complications.

10. The dendrimer complex for use according to any one of claims 1-9, wherein the dendrimer complex is administered in a dosage between about 0.01 mg/kg and about 100 mg/kg body weight of the subject.

11. A dendrimer complex for use in a method for diagnosing an inflammatory gastrointestinal disorder, the method comprising orally administering to a subject a dendrimer complex consisting of a poly(amidoamine) (PAMAM) hydroxyl-terminated dendrimer having one or more diagnostic agents conjugated to the dendrimer, wherein the dendrimer complex crosses the blood brain barrier, wherein the diagnostic agent is a fluorophore, wherein the use is in a subject that has inflammation in the gastrointestinal tract and/or a disorder with central nervous system complications.

12. The dendrimer complex for use according to claim 11, wherein the PAMAM dendrimer is a G3, G4, G5, or G6 PAMAM dendrimer.

13. The dendrimer complex for use according to claim 11, wherein the diagnostic agent is indocyanine green.

14. The dendrimer complex for use according to any one of claims 1-13, wherein the dendrimers are formulated in a suspension, emulsion, tablet, capsule, or lavage.

15. The dendrimer complex for use according to any of one claims 1-14, wherein the dendrimers are formulated in a formula for infants.

## Patentansprüche

1. Dendrimerkomplex zur Verwendung in einem Verfahren zur Behandlung einer entzündlichen gastrointestinalen Störung, das Verfahren umfassend die orale Verabreichung eines Dendrimerkomplexes an ein Subjekt, bestehend aus einem Poly(amidoamin)-(PAMAM)-Hydroxyl-terminierten Dendrimer mit einem oder mehreren entzündungshemmenden Mitteln, die an das Dendrimer konjugiert sind, wobei der Dendrimerkomplex die Blut-Hirn-Schranke durchquert, wobei die Verwendung bei einem Subjekt erfolgt, das eine Entzündung im Gastrointestinaltrakt und/oder eine Störung mit Komplikationen im zentralen Nervensystem aufweist.

2. Dendrimerkomplex zur Verwendung nach Anspruch 1, wobei das PAMAM-Dendrimer ein G3-, G4-, G5- oder G6-PAMAM-Dendrimer ist.

3. Dendrimerkomplex zur Verwendung nach einem der Ansprüche 1 bis 2, wobei das PAMAM-Dendrimer über eine Disulfidbindung mit dem einen oder mehreren entzündungshemmenden Mitteln verbunden ist.

4. Dendrimerkomplex zur Verwendung nach einem der Ansprüche 1 bis 3, wobei das PAMAM-Dendrimer über eine oder mehrere Abstandshalterverbindungen, ausgewählt aus der Gruppe, bestehend aus N-Succinimidyl-3-(2-pyridyldithio)propionat (SPDP), Glutathion (GSH), Gamma-Aminobuttersäure (GABA) und Kombinationen davon, mit dem einen oder mehreren entzündungshemmenden Mitteln verbunden ist.

5. Dendrimerkomplex zur Verwendung nach einem der Ansprüche 1 bis 4, wobei mindestens einer der einen oder mehreren entzündungshemmenden Mittel N-Acetylcystein ist.

6. Dendrimerkomplex zur Verwendung nach einem der Ansprüche 1 bis 4, wobei mindestens einer der einen oder mehreren entzündungshemmenden Mittel ein Inhibitor des Toll-ähnlichen Rezeptors 4 ist.

7. Dendrimerkomplex zur Verwendung nach einem der Ansprüche 1 bis 6, wobei die zu behandelnde Störung eine nekrotisierende Enterokolitis (NEC) ist.

8. Dendrimerkomplex zur Verwendung nach einem der Ansprüche 1 bis 6, wobei die zu behandelnde Störung ausgewählt ist aus der Gruppe, bestehend aus abdominaler Sepsis und Pankreatitis.

9. Dendrimerkomplex zur Verwendung nach einem der Ansprüche 1 bis 6, wobei das Subjekt eine Entzündung im Magen-Darm-Trakt und eine Störung mit Komplikationen im zentralen Nervensystem aufweist.

10. Dendrimerkomplex zur Verwendung nach einem der Ansprüche 1 bis 9, wobei der Dendrimerkomplex in einer Dosierung zwischen etwa 0,01 mg/kg und etwa 100 mg/kg Körpergewicht des Subjekts verabreicht wird.

11. Dendrimerkomplex zur Verwendung in einem Verfahren zur Diagnose einer entzündlichen gastrointestinalen Störung, das Verfahren umfassend die orale Verabreichung eines Dendrimerkomplexes an ein Subjekt, bestehend aus einem Poly(amidoamin)-(PAMAM)-hydroxyl-terminierten Dendrimer mit einem oder mehreren an das Dendrimer konjugierten diagnostischen Mitteln, wobei der Dendrimerkomplex die Blut-Hirn-Schranke überwindet, wobei das diagnostische Mittel ein Fluorophor ist, wobei die Verwendung bei einem Subjekt erfolgt, das eine Entzündung im Gastrointestinaltrakt und/oder eine Störung mit Komplikationen des zentralen Nervensystems aufweist.

12. Dendrimerkomplex zur Verwendung nach Anspruch 11, wobei das PAMAM-Dendrimer ein G3-, G4-, G5- oder G6-PAMAM-Dendrimer ist.

13. Dendrimerkomplex zur Verwendung nach Anspruch 11, wobei das diagnostische Mittel Indocyaningrün ist.

14. Dendrimerkomplex zur Verwendung nach einem der Ansprüche 1 bis 13, wobei die Dendrimere in einer Suspension, Emulsion, Tablette, Kapsel oder Lavage formuliert sind.

15. Dendrimerkomplex zur Verwendung nach einem der Ansprüche 1 bis 14, wobei die Dendrimere in einer Formel für Säuglinge formuliert sind.

## Revendications

1. Complexe dendrimère destiné à être utilisé dans un procédé de traitement d'un trouble gastro-intestinal inflammatoire, le procédé comprenant l'administration par voie orale à un sujet d'un complexe dendrimère constitué d'un dendrimère à terminaison hydroxyle poly(amidoamine) (PAMAM) présentant un ou plusieurs agents anti-inflammatoires conjugués au dendrimère, dans lequel le complexe dendrimère traverse la barrière hémato-encéphalique, dans lequel l'utilisation est effectuée chez un sujet présentant une inflammation du tractus gastro-intestinal et/ou un trouble avec des complications du système nerveux central.

2. Complexe dendrimère destiné à être utilisé selon la revendication 1, dans lequel le dendrimère PAMAM est un dendrimère PAMAM G3, G4, G5 ou G6.

3. Complexe dendrimère destiné à être utilisé selon l'une quelconque des revendications 1 et 2, dans lequel le dendrimère PAMAM est lié aux un ou plusieurs agents anti-inflammatoires par l'intermédiaire d'une liaison disulfure.

4. Complexe dendrimère destiné à être utilisé selon l'une quelconque des revendications 1 à 3, dans lequel le dendrimère PAMAM est lié aux un ou plusieurs agents anti-inflammatoires par l'intermédiaire d'un ou plusieurs composés espaceurs sélectionnés dans le groupe constitué du 3-(2-pyridyldithio)propionate N-succinimidyle (SPDP), du glutathion (GSH), de l'acide gamma-aminobutyrique (GABA) et de combinaisons ceux-ci.

5. Complexe dendrimère destiné à être utilisé selon l'une quelconque des revendications 1 à 4, dans lequel au moins un des un ou plusieurs agents anti-inflammatoires est la N-acétylcystéine.

6. Complexe dendrimère destiné à être utilisé selon l'une quelconque des revendications 1 à 4, dans lequel au moins un des un ou plusieurs agents anti-inflammatoires est un inhibiteur du récepteur de type Toll 4.

7. Complexe dendrimère destiné à être utilisé selon l'une quelconque des revendications 1 à 6, dans lequel le trouble à traiter est l'entérocolite nécrosante (ECN).

8. Complexe dendrimère destiné à être utilisé selon l'une quelconque des revendications 1 à 6, dans lequel le trouble à traiter est choisi dans le groupe constitué de la septicémie abdominale et de la pancréatite.

9. Complexe dendrimère destiné à être utilisé selon l'une quelconque des revendications 1 à 6, dans lequel le sujet présente une inflammation du tractus gastro-intestinal et un trouble avec des complications du système nerveux central.

10. Complexe dendrimère destiné à être utilisé selon l'une quelconque des revendications 1 à 9, dans lequel le complexe dendrimère est administré à une dose comprise entre environ 0,01 mg/kg et environ 100 mg/kg de poids corporel du sujet.

11. Complexe dendrimère destiné à être utilisé dans un procédé de diagnostic d'un trouble gastro-intestinal inflammatoire, le procédé comprenant l'administration par voie orale à un sujet d'un complexe dendrimère constitué d'un dendrimère à terminaison hydroxyle poly(amidoamine) (PAMAM) présentant un ou plusieurs agents de diagnostic conjugués au dendrimère, dans lequel le complexe dendrimère traverse la barrière hémato-encéphalique, dans lequel l'agent de diagnostic est un fluorophore, dans lequel l'utilisation est effectuée chez un sujet qui présente une inflammation du tractus gastro-intestinal et/ou un trouble avec des complications du système nerveux central.

12. Complexe dendrimère destiné à être utilisé selon la revendication 11, dans lequel le dendrimère PAMAM est un dendrimère PAMAM G3, G4, G5 ou G6.

13. Complexe dendrimère destiné à être utilisé selon la revendication 11, dans lequel l'agent de diagnostic est le vert d'indocyanine.

14. Complexe de dendrimère destiné à être utilisé selon l'une quelconque des revendications 1 à 13, dans lequel les dendrimères sont formulés dans une suspension, une émulsion, un comprimé, une capsule ou un lavage.

15. Complexe de dendrimère destiné à être utilisé selon l'une quelconque des revendications 1 à 14, dans lequel les dendrimères sont formulés dans une formule pour nourrissons.
